# EUROPEAN PATENT SPECIFICATION

(11) **EP 3 556 298 B1**
(45) Date of publication and mention of the grant of the patent: **09.12.2026**
(21) Application number: 19177060.1
(22) Date of filing: 11.10.2010
(51) Int. Cl.: A61B 17/00

(54) **SINGLE INCISION LAPAROSCOPIC TISSUE RETRIEVAL SYSTEM**
LAPAROSKOPISCHES SYSTEM FÜR EINZELINZISIONSGEWEBEENTNAHME
SYSTÈME DE RÉCUPÉRATION DE TISSUS DE LAPAROSCOPIE À INCISION UNIQUE

(30) Priority: 09.10.2009 US 25036409 P; 01.10.2010 US 38910110 P
(43) Date of publication of application: 23.10.2019
(60) Divisional application: 26211853.2 / 4 846 946
(62) Document number(s) of the earlier application(s) in accordance with Art. 76 EPC: 10771261.4 / 2 485 652
(73) Proprietor: Applied Medical Resources Corporation, Rancho Santa Margarita, CA 92688 (US)
(72) Inventor: Taylor, Scott V, Rancho Santa Margarita, CA 92688 (US); Kahle, Henry, Rancho Santa Margarita, CA 92688 (US)
(74) Representative: Dolleymores

(56) References cited:
- EP-A1- 1 679 040
- EP-A1- 1 707 126
- WO-A1-96/16601
- WO-A2-2008/114234
- US-A- 5 735 289
- US-B1- 6 409 733

## Description

### FIELD OF THE INVENTION

The present application relates to a tissue retrieval system for capturing and retrieving tissue from body cavities and in particular relates to a specimen retrieval bag device.

### DESCRIPTION OF THE RELATED ART

Laparoscopic surgery is typically performed through trocars, which provide access across the abdominal wall and into the abdominal cavity. In some of surgeries, tissue disposed within the abdominal cavity is cut and removed from the body. However, removal of such tissue from the body may prove difficult due to the limited confines inherent with laparoscopic surgery and the available laparoscopic surgical instruments. For example, to reduce the invasiveness to a patient, it can be desirable to introduce all of the surgical instruments through a single laparoscopic port having a relatively small size. Also, removed tissue may include an infected or cancerous mass or organ, as well as blood, bile and other liquids, all referred to herein as tissue, which may pose infection issues or other complications if left within the body.

It is desirable to grasp, capture, retain and enclose this tissue while in the body cavity, and then remove the enclosed tissue through the trocar or incision. Containment of the tissue as quickly as possible with minimal disturbance to the surgical site is also desirable. A generally compact and single unit device would also prove desirable as devices generally bulky and complicated have several shortcomings and lack optimal efficiency in particular with the limited space in operating rooms and access ports in the body cavity.

European Patent Application, publication number 1707126 A1, US patent publication number US 6,409,733 B1 and International Patent Application, publication number 2008/114234 A2 disclose known types of tissue retrieval systems.

### SUMMARY OF THE INVENTION

In accordance with the present invention there is provided a tissue retrieval system as recited in Claim 1 of the claims of this specification.

### BRIEF DESCRIPTION OF THE DRAWINGS

Figure 1 is an isometric view of a tissue retrieval system in accordance with the present invention;
Figure 2 is a side view of the tissue retrieval system of Figure 1 with the retrieval bag deployed;
Figure 3 is a top view of the tissue retrieval system of Figure 1 with the retrieval bag deployed;
Figure 4A is a side view of the tissue retrieval bag of the tissue retrieval system of Figure 1;
Figure 4B is a side view of a guide bead for the tissue retrieval bag of the tissue retrieval system of Figure 1;
Figure 4C is a front view of the introducer tube of the tissue retrieval system of Figure 1;
Figure 4D is a partial cut away view of the introducer tube of Figure 4C;
Figure 5 is a side view of one support arm of the tissue retrieval system of Figure 1;
Figure 6 is a top view of the support arm of Figure 5;
Figure 7 is a top view of a tissue retrieval bag 20', of a tissue retrieval system not in accordance with the present invention, having an elongate profile and hybrid construction in a partially-formed state;
Figure 8 is a side view of the tissue retrieval bag of Figure 7 in a fully-formed state;
Figure 9 is a top view of a cuff reinforcement tab for the tissue retrieval bag of Figures 7-8;
Figure 10 is a perspective view of a tissue retrieval system not in accordance with the present invention, including a hybrid tissue retrieval bag;
Figure 11 is a side view of a first hybrid tissue retrieval bag of a tissue retrieval system not in accordance with the present invention;
Figure 12 is a side view of a second hybrid tissue retrieval bag of a tissue retrieval system not in accordance with the present invention;
Figure 13 is a side view of a third hybrid tissue retrieval bag of a tissue retrieval system not in accordance with the present invention;
Figure 14 is a side view of a fourth hybrid tissue retrieval bag of a tissue retrieval system not in accordance with the present invention; and
Figure 15 is a side view of a fifth hybrid tissue retrieval bag of a tissue retrieval system not in accordance with the present invention.

### DETAILED DESCRIPTION OF THE INVENTION

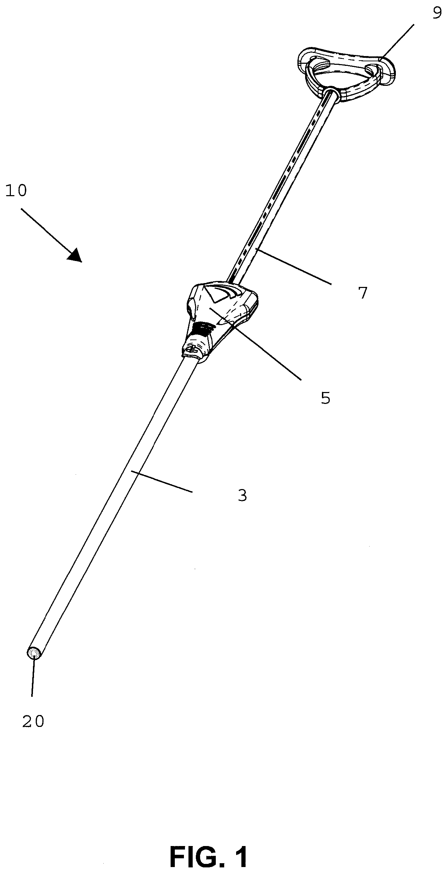

With reference to Figure 1, there is illustrated a tissue retrieval system 10 in accordance with the present invention. The illustrated tissue retrieval system can be used for containing and withdrawing excised tissue specimens from within a body cavity. For example, the tissue retrieval system may be used to remove a patient's gallbladder from the patient's abdominal cavity. Thus, advantageously, the tissue retrieval systems discussed herein provides an easy-to-use tissue retrieval system which effectively contains excised tissue specimens to prevent loss or spillage of tissue specimens into a body cavity, and to protect the body wall access port site from contamination with the excised tissue specimens during withdrawal of the tissue specimens from within the body cavity.

With continued reference to Figure 1, the tissue retrieval system 10 is illustrated in a non-deployed or non-activated initial condition. The tissue retrieval system has an introducer 3 and an actuator or actuation rod 7. The introducer 3 has a tubular configuration with a hollow lumen and a handle assembly 5 extending from a proximal end of the introducer 3. The introducer 3 can be sized and configured for placement through a standard-size trocar. For example, it can be desirable that the introducer 3 can be sized as a 5 mm laparoscopic surgical instrument to be introduced through relatively small diameter trocars such as 5-7mm trocars. Alternatively, the introducer 3 can be sized as a 10 mm laparoscopic surgical instrument. The introducer 3 can though have a non-standard size for application at a specific location. The tissue retrieval system 10 can include a relatively long introducer, such as, for example, a 45cm long introducer 3 to improve access to the surgical site.

The handle assembly 5 can comprise a compact handle member that can be adapted for placement adjacent other surgical instruments in a single port laparoscopic surgical site. Thus, the tissue retrieval system can be adapted to be utilized during single incision laparoscopic procedures. Alternatively, the handle assembly can include a pair of finger loops or grips formed with or otherwise coupled to the handle assembly 5 that can be utilized to hold or stabilize the introducer 3 as desired.

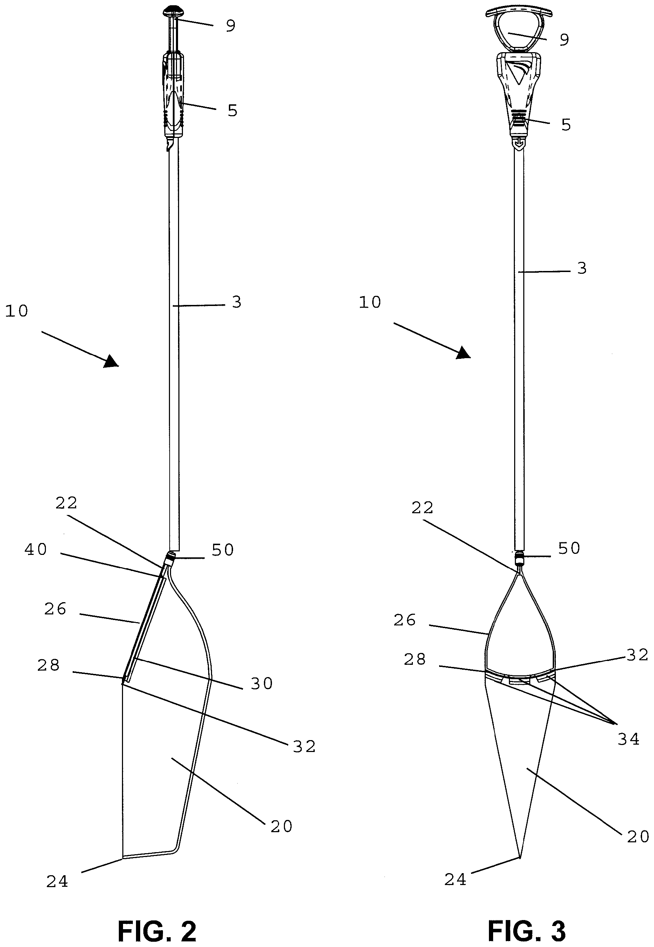

The introducer 3 has a proximal end and a distal end that are generally open, to facilitate access to the hollow lumen. Actuator rod 7 extends into the hollow lumen from the open proximal end thereof, and at least a portion of the actuator rod 7 is slidably movable within the hollow lumen of the introducer 3. As further discussed with reference to Figures 2 and 3, with the tissue retrieval system 10 in the initial configuration, a tissue retrieval bag 20 in a stowed configuration is positioned in the hollow lumen of the introducer 3. The actuator rod 7 has a handle 9 extending from a proximal end thereof. The handle 9 provides a graspable portion of the device to control or facilitate movement of the actuator rod 7 relative to the introducer 3 between the initial condition of the tissue retrieval system 10 (illustrated in Figure 1), and a deployed condition of the tissue retrieval system (illustrated in Figures 2-3).

With reference to Figures 2 and 3, the tissue retrieval system includes a retrieval bag 20 that is deployable from the distal end of the introducer 3 which can be used as a receptacle for tissue specimens. After insertion of tissue specimens into the retrieval bag 20, the retrieval bag can then be cinched closed to prevent spillage of its contents and to prevent contamination of the body cavity and body cavity wall during withdrawal of the retrieval bag 20 from within the body cavity.

With reference to Figures 2, 3, and 4A, here the tissue retrieval system includes a retrieval bag 20 sized and configured to be contained within a relatively small (such as, for example, a 5-7mm) diameter introducer tube while providing a retrieval bag with a similar size and volume as laparoscopic retrieval bags for use with 10mm trocars. Advantageously, such a retrieval bag 20 can facilitate various laparoscopic surgical procedures with the use of relatively small single incision site surgical access points.

With continued reference to Figures 2, 3, and 4A, the tissue retrieval bag 20 has a distal end 24 opposite a proximal end 22. The tissue retrieval bag 20 includes a rim 26 defining an opening adjacent the proximal end 22 into the tissue retrieval bag 20, while the distal end 24 of the tissue retrieval bag 20 is closed. Thus, the tissue retrieval bag 20 has an elongate profile, with a portion of the tissue retrieval bag 20 extending distally from the rim 26 and the opening. The distally extending portion of the retrieval bag diverges at an angle transverse to the rim 26 of the bag 20.

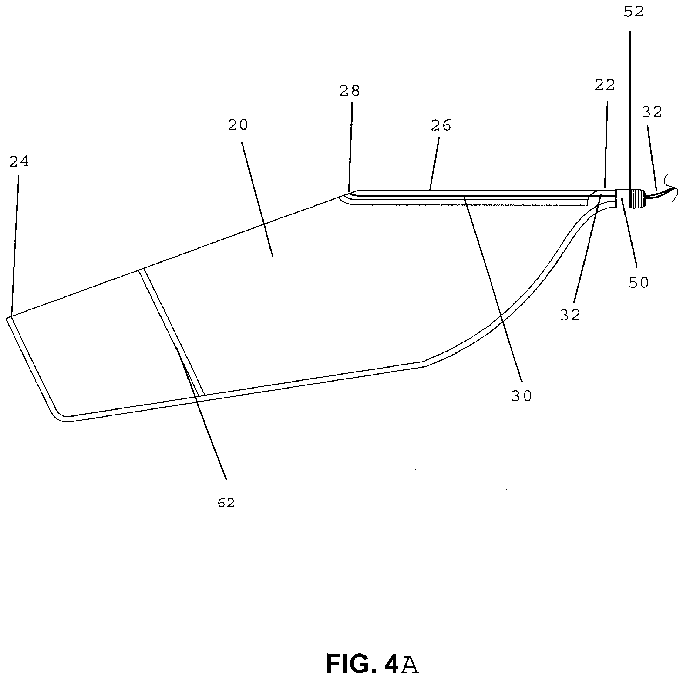

This elongate profile allows the tissue retrieval bag 20 to have a relatively small outer diameter when in a stowed configuration (Figure 1), while having a relatively large volume in a deployed configuration (Figures 2, 3, and 4A). Thus, the tissue retrieval bag 20, in the stowed configuration, can fit within a relatively small diameter introducer tube 3. With reference to Figure 4A, in order to position the tissue retrieval bag 20 in the stowed configuration, the tissue retrieval bag 20 can be rolled about an axis generally parallel to a longitudinal axis defined by the rim 26 and the opening of the bag. Thus, in the stowed configuration, the distal end 24, and the distal-extending portion of the tissue retrieval bag 20 is positioned longitudinally distally of the rim 26 and the opening with respect to the axis defined by the rim 26. The tissue retrieval bag 20 in the stowed configuration can be stored within the introducer tube 3. The cross-sectional area at any point along the length of the rolled retrieval bag with respect to the longitudinal axis defined by the rim 26 is equivalent to or smaller than the cross-sectional area of a relatively small introducer tube, such as a 5-7mm introducer tube. Likewise, the cross-sectional area at any point along the axis of the rolled retrieval bag 20 is decreased as compared to prior retrieval bags of approximately the same volume.

The tissue retrieval bag 20 can be sized such that the volume of the retrieval bag is approximately 180 mL and wherein the tissue retrieval bag 20, in the stowed configuration, fits within a 5-7 mm trocar introducer. Accordingly, a ratio of the volume of the retrieval bag to its stowed diameter can be relatively high, in the example, approximately 26-36 mL/mm. In other examples, the tissue retrieval bag 20 can be sized such that the volume is between about 50 mL and 400 mL, desirably between about 100 mL and 350 mL, and more desirably between about 150 mL and 200 ml. The volume of the retrieval bag 20, to fit within a predetermined size trocar, can be increased further by further elongating the bag to enable a greater amount of tissue to be placed within the bag. For example, a tissue retrieval bag for placement within a 5-7 mm trocar introducer can have a first length between the proximal end 22 and the distal end 24 and a first volume, while other examples of tissue retrieval bag for placement within a 5-7 mm trocar introducer can have a second length between the proximal end 22 and the distal end 24 and a second volume, where the first length is smaller than the second length and the first volume is smaller than the second volume.

The retrieval bag 20 can be configured from 0.11mm (4.2 mil) thick polyurethane film, but the thickness of the polyurethane film can be greater than or less than 0.11 mm (4.2 mil), such as, for example for use in extracting tissue of a relatively high or low weight. The retrieval bag can be formed from a variety of materials including polyurethane, polyethylene, polyimide, ripstop Nylon^{®}, polyester, and Mylar^{®}. The retrieval bag can also be formed from laminated materials such as polyurethane coated ripstop Nylon, silicone coated ripstop Nylon, polyurethane coated ripstop polyester, silicone coated ripstop polyester, polyurethane coated taffeta, and polyurethane coated Spandex^{®}. The thicknesses of any of these materials can be chosen based, at least partially, on considerations of tissue weight to be carried by the tissue retrieval bag and outer diameter of the tissue retrieval bag in a stowed configuration.

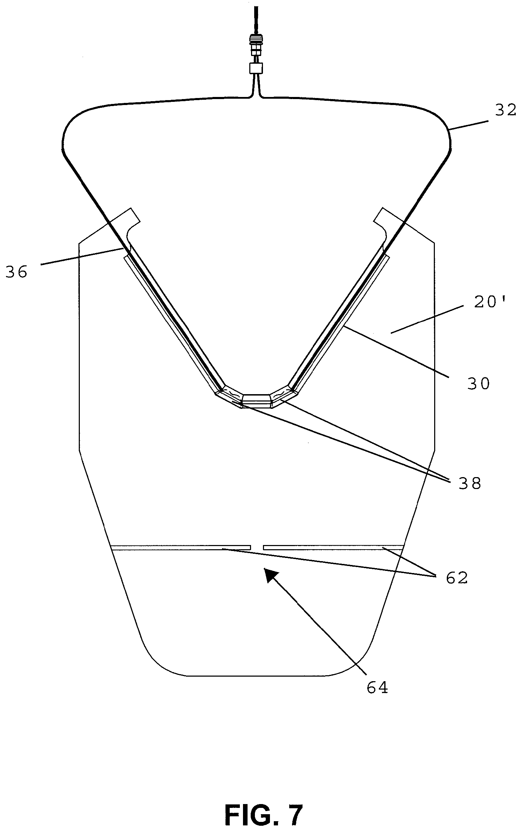

As further discussed below with respect to Figures 7-15, the tissue retrieval bag 20 can have hybrid properties-that is material properties that vary from the open end of the bag to the closed end of the bag. For example, the tissue retrieval bag 20 can be formed with two different thicknesses of the same film material. For example, a portion of the tissue retrieval bag 20 adjacent the distal end of the retrieval bag can be formed with a relatively thick film and have a relatively low compliance while the remainder of the retrieval bag can be formed with a thinner film and have a relatively higher compliance. By incorporating a film with an increased thickness in the distal portion of the retrieval bag, the tensile and burst strengths of the retrieval bag can be increased, or a tissue retrieval bag having equivalent tensile and burst strengths can have a smaller outer diameter in the stowed configuration.

The tissue retrieval bag can be formed with two different materials. For example, a portion of the tissue retrieval bag adjacent the distal end can be formed from one material having a relatively high tensile strength while the remainder of the retrieval bag can be formed from a different material having a relatively lower tensile strength. For example, the distal portion of the retrieval bag can be formed from a polyurethane and ripstop Nylon laminate while the remainder of the retrieval bag can be formed from a polyurethane film. The material utilized for the distal tip of the retrieval bag can have an increased thickness as compared to the material utilized for the remainder of the retrieval bag. By incorporating different materials in different areas of the retrieval bag, the tensile and burst strengths of the retrieval bag can be increased, or a tissue retrieval bag having equivalent tensile and burst strengths can have a smaller outer diameter in the stowed configuration.

With reference to Figures 2, 3, and 4A, the tissue retrieval bag includes a non-continuous cuff 30 for retaining a cord 32 that is used for cinching the retrieval bag closed and for withdrawing the retrieval bag 20 through a body wall. During withdrawal of the retrieval bag 20 through a body wall, tensile stresses are concentrated at the distal portion 28 of the rim 26. This concentration of stresses can be especially pronounced in the withdrawal of the retrieval bag 20 through a relatively small diameter incision or access device. Therefore, it can be desirable to provide the retrieval bag 20 with a reinforced distal cuff 30 having a reinforced section at a distal portion 28 of the rim 26 or opening. The cuff 30 can include one or more loops 34 formed at the distal portion 28 of the rim 26 for retaining the cord 32. The loop 34 can consist of a single layer of film, or can comprise multiple layers of film to provide the retrieval bag with a reinforced cuff. The number of layers and thickness of each layer can be sized to provide a predetermined tensile strength for the retrieval bag.

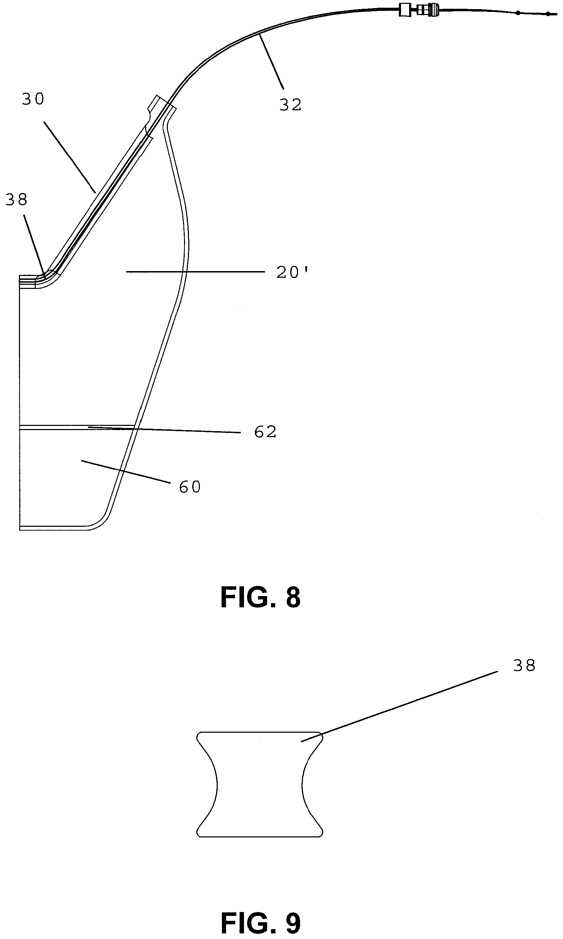

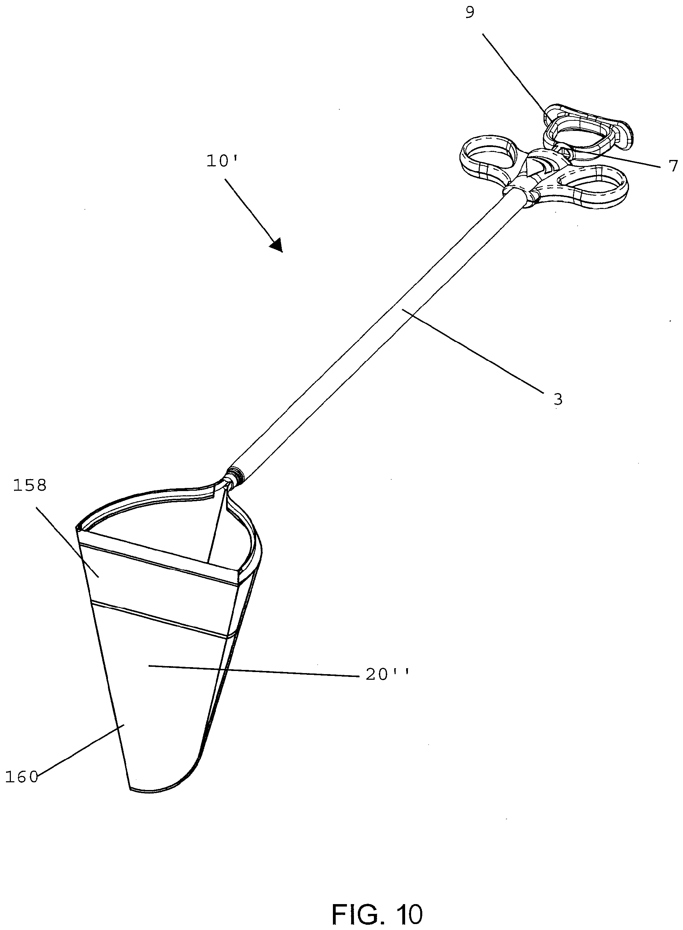

With reference to Figures 7-9, an alternative tissue retrieval bag 20', not in accordance with the present invention, is illustrated having several features of the above-described tissue retrieval bags. Figure 7 illustrates the tissue retrieval bag 20' in a partially formed state, illustrating partial assembly of the bag from a flat sheet of a film before a closed end of the bag has been formed such as by welding. Figure 8 illustrates the tissue retrieval bag 20' in an assembled state with a sealed closed end. The tissue retrieval bag 20' includes a distal end that has been reinforced with a reinforcing section 60 joined to the remainder of the bag 20' at a seam 62. For example, the retrieval bag 20' can comprise a 0.10mm (4 mil) film, and the reinforcing section can comprise, for example, a second layer of film material having a thickness of 0.05mm (2 mil) for a total 0.15mm (6 mil) thickness at the reinforcing section. As discussed in further detail with respect to Figures 10-13, the seam 62 can include a space or gap 64 therein to provide a vented cavity between the reinforcing section 60 and ambient conditions.

With continued reference to Figures 7-9, a cord loop 32 passes through a cuff 30 at an opening 36 in the cuff 32. The cuff 30 can be formed in a flat sheet of film material by folding a portion of the film material over itself and joining the folded portion to the remainder of the material such as by welding. Desirably, the cuff 30 can be formed with the cord loop 32 in place. In this arrangement, not in accordance with the present invention, the cuff is substantially continuous, as it includes a plurality of reinforcing tabs 38 joined to the cuff 30 to provide enhanced strength for a portion of the cuff encountering a relatively high tensile stress during use. Desirably, at least one reinforcing tab 38 can be added to the cuff 30 to form a reinforced section of the cuff 30. In the illustrated example, two reinforcing tabs 38 are joined to the cuff 30 to reinforced the distal-most end of the cuff. Alternatively, more or fewer than 2 reinforcing tabs can be added to the cuff to form a cuff having the desired level of continuity and reinforcement.

A detail view of one reinforcing tab 38 is illustrated in Figure 9. The reinforcing tab 38 is formed from a segment of a film material having a substantially hourglass shape. Advantageously, such a geometry can facilitate folding and rolling of the tissue retrieval bag 20' to a relatively low diameter configuration for installation in a trocar. However, the cuff tab can have different geometries, such as substantially rectangular or oval geometries to create a cuff having desired strength and stowage properties.

Certain aspects of the retrieval bag described herein for use with 5-7mm trocars can also be adapted to be used with 10mm and larger diameter trocars. In this case, the diameter of the introducer tube would be approximately 10mm, such as, for example, 10-12mm. For example, a tissue retrieval system with an approximately 10mm introducer tube can include a tissue retrieval bag having elongate profile comprising a film thickness of approximately 0.20mm (8 mils). Advantageously, such a tissue retrieval bag with an elongate profile has enhanced tensile and burst strength characteristics as compared to prior 10mm retrieval bags.

Alternatively, the tissue retrieval system can be for use with 10 mm and larger diameter trocars, where a tissue retrieval bag can be configured to have a relatively large volume, rather than enhanced strength as compared to prior 10 mm retrieval bags. For example, an elongate profile of a tissue retrieval bag configured for use with 5-7mm trocars can be generally scaled up in size, but not film thickness, to be used with 10mm and larger diameter trocars. Thus, the resulting tissue retrieval bag would fit within a 10mm introducer tube and have an increased volume relative to prior 10mm tissue retrieval bags to accommodate a large tissue specimen, such as a spleen. The dimensions and film thickness of a tissue retrieval bag for use with a 10 mm or larger diameter trocar can each be selected such that the tissue retrieval bag has desired volume and strength characteristics.

The tissue retrieval system could be provided with a 15mm introducer tube and incorporate a larger version of a tissue retrieval bag having the elongate profile of the 5mm retrieval bag described above. The 15mm retrieval bag could also incorporate film materials with greater thicknesses than those utilized for the 5mm retrieval bags, or some combination of an elongate profile and varied film thickness.

With reference to Figures 2, 5, and 6, the tissue retrieval systems 10 described herein includes retrieval bag support arms 40 which are biased to a predetermined position when the tissue retrieval system is in the deployed configuration. The support arms 40 are biased to spring radially outward and transversely upward with respect to a longitudinal axis defined by the introducer 3 upon deployment of the retrieval bag 20 from the introducer 3. Desirably, by springing transversely upward upon deployment of the retrieval bag 20, the support arms create an access position having an angled entry for the rim 26 of the retrieval bag 20 relative to the axis of the introducer 3. Advantageously, the angled entry facilitates the loading of tissue specimens during laparoscopic procedures as it allows a grasped tissue sample to be easily disposed within the retrieval bag 20. This ease of loading can be particularly advantageous for single incision laparoscopic procedures where the grasped tissue is held by a grasper that is disposed through the same incision as the retrieval system introducer 3. Thus, the transverse orientation of the retrieval bag 20 rim 26 relative to the longitudinal axis of the introducer 3 enables a tissue specimen held by a grasper to be easily inserted and pushed towards the distal end 24 of the retrieval bag 20 during single incision laparoscopic procedures where it would otherwise be particularly difficult to create a substantial axial angle between the grasper shaft and the introducer tube.

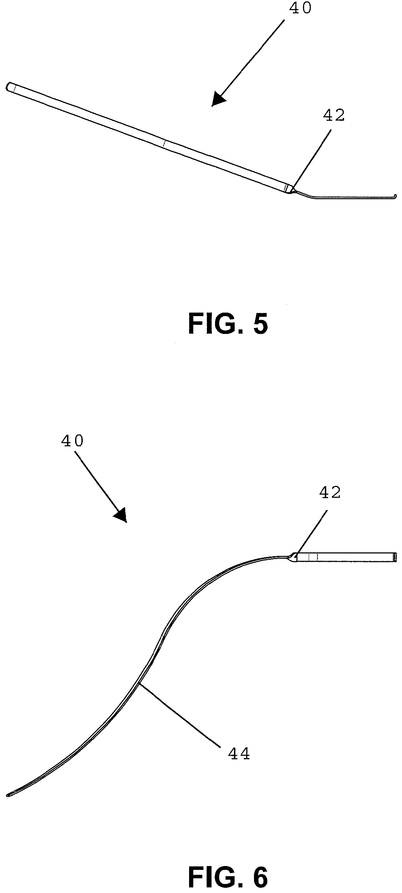

As illustrated, the support arms 40 can be bent to include upward bends 42 in each support arm 40 in the sections of the support arms that are proximal to where a bead 50 is positioned and distal to the end of the actuation rod. When viewed from a top perspective, as illustrated in Figure 6, each of the support arms 40 can also include a radial bend, curve, or curved profile 44. As the retrieval bag 20 is advanced out of the introducer 3, the support arms 40 spring radially outward to return to the radial curved profile 44, thus opening the retrieval bag. As the retrieval bag 20 is further advanced out of the introducer 3, the support arms 40 spring upward about the bends 42 in the support arms 40 to position the retrieval bag 20 in the access position with an angled opening relative to the longitudinal axis of the introducer 3. As the retrieval bag 20 is cinched closed, the support arms 40 flex downward and radially inward as they are retracted into the introducer 3.

The support arms 40 can be formed from 17-7PH stainless steel, which is a typical spring metal or can comprise other metals and metal alloys having desired biasing properties. The support arms 40 can be formed of a memory metal such as a nickel titanium alloy or nitinol metal. The memory metal can be preshaped to be generally linear in the introducer 3 with the tissue retrieval system in the initial configuration and to form a radially expanded and transversely bent shape upon application of body heat to the support arm 40 as the tissue retrieval bag 20 is deployed into a body cavity.

Instead of, or in addition to the biased support arms 40 discussed above, the support arms could instead pivot about a pin and the tissue retrieval system can include a torsion spring to drive the support arms upward upon deployment from the introducer tube. This spring-biased pivot mechanism can provide the retrieval bag with an angled opening relative to the longitudinal axis of the introducer tube.

During clinical use of the tissue retrieval systems described herein, an access device such as a trocar is first placed through a body wall leaving the trocar cannula disposed across the body wall. It is contemplated that a wound retractor, such as an Alexis^{®} wound retractor can be positioned in an incision in the patient's body before placement of the trocar cannula. Here, a wound retractor is initially inserted into an incision. Then, any adjustments to the length or diameter of the wound retractor are made. For example, with certain wound retractors, an external ring of the retractor can be rolled to remove slack in an elastomeric sheet forming the wound retraction surface.

The tissue retrieval system 10 is then inserted into the trocar seal and cannula until the distal end of the introducer 3 extends beyond the distal end of the trocar cannula. The retrieval bag 20 is then deployed from within the introducer 3 and into the body cavity by pushing the actuator 7 in a distal direction relative to the introducer 3. The actuator rod can include a ratcheting mechanism which allows distal movement of the actuator 7 during retrieval bag deployment and prevents the actuator 7 from being pulled in a proximal direction to ensure proper operation of the device. Once extended into the body cavity, the retrieval bag 20 is suspended and held open by two support arms 40 that extend into the cuff 30 on the retrieval bag 20.

The retrieval bag 20 can include an integral bead 50 through which the support arms 40 can slide. The retrieval bag 20 also includes a cord loop 32 which runs through the cuff 30 of the retrieval bag 20 and is releasably attached to the actuator 7. The bead 50 can include a frictional lock through which the cord loop 32 runs to enable the retrieval bag 20 to be cinched closed and reopened as needed.

With reference to FIG. 4A, the bead 50 can include an expandable, relatively large diameter member such as a snap ring 52 positioned about a periphery thereof. The snap ring 52 can prevent reentry of the guide bead 50 into the introducer 3 once the actuator 7 has advanced the retrieval bag 20 and guide bead 50 out of the introducer, thus facilitating cinching of the retrieval bag 20 when the cord 32 is pulled. A portion of bag material adjacent the guide bead 50 can facilitate cinching of the retrieval bag 20. For example, the guide bead 50 can be pulled within the introducer 3 tube while the tissue retrieval bag 20 can collect and bunch up outside the introducer 3 tube when the cord is pulled.

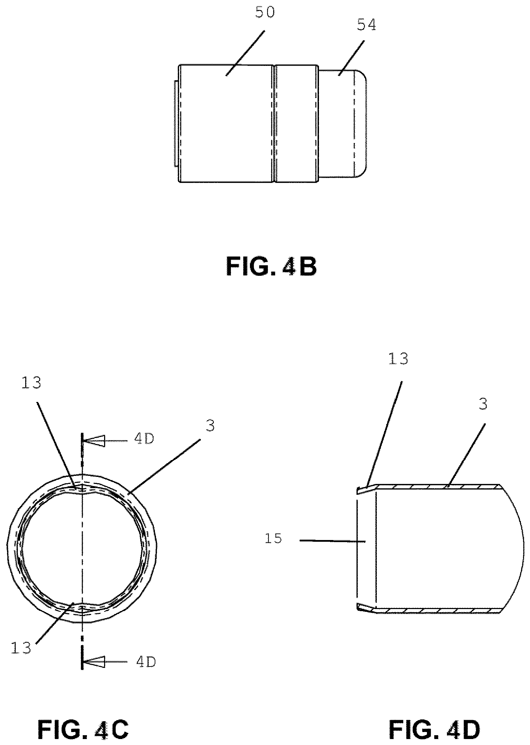

With reference to FIGS. 4B, 4C, and 4D, introducer 3 can have a distal end configured to allow passage of the guide bead 50 during deployment of the retrieval bag 20 while also restricting reentry of the deployed guide bead 50 into the introducer 3. The distal end of the introducer 3 can comprise at least one restrictor such as one or more generally vee-shaped ramped surfaces 13. The introducer 3 includes two generally diametrically opposed ramped surfaces 13. The introducer 3 can have more or fewer than two ramped surfaces, and the ramped surfaces can be generally regularly spaced about the periphery, or can be irregularly spaced. The ramped surfaces can define a gradually decreasing inner diameter of the introducer 3 to a relatively low diameter region 15 minimum inner diameter at the distal end. Desirably, the ramped surfaces 13 extend a relatively short distance in a longitudinal direction such that the ramped surfaces 13 do not interfere with movement of the actuator 7. The guide bead 50 can include an interface surface, such as a diametric step 54 to interface with ramped surfaces 13 of the introducer 7. Advantageously, the ramped surfaces 13 facilitate easier passage of the rolled retrieval bag 20 through the introducer 3 while preventing reentry of the guide bead 50 to cinch the retrieval bag 20.

Once a tissue specimen such as a gallbladder is separated from the adjoining vessels and structures, it can then be placed into the retrieval bag 20. The actuator 7 is then pulled proximally to withdraw the support arms 40 from the cuff 30 and the bead 50 on the retrieval bag 20. The actuator 7 can include a ratcheting mechanism which allows proximal movement of the actuator 7 during cinching of the retrieval bag 20 and prevents distal movement of the actuator 7 to ensure proper and complete closure of the retrieval bag 20. As the support arms 40 are pulled out of the cuff 30 of the retrieval bag 20 and through the bead 50, tension is then applied to the cord loop 32 to cinch the bag 20 closed. Once the retrieval bag 20 is fully closed, a small loop of the cord 32 is exposed on the actuator 7 near the proximal end of the introducer 3.

At this stage, there are at least three methods for withdrawal of the retrieval bag 20 from within the body cavity. For one method, the retrieval bag 20 can be completely detached and removed from the actuator 7 and introducer 3 by lifting the cord loop 32 from a retaining feature such as a retaining slot on the actuator 7. The device and the trocar seal and cannula can then be withdrawn from the body wall leaving the retrieval bag 20 in the body cavity and the cord loop 32 disposed across the body wall. The neck of the retrieval bag 20 can then be withdrawn through the body wall using the bead 50 as a dilator to aid with movement of the retrieval bag through the layers of tissue fibers in the body wall. Once the neck of the retrieval bag 20 has traversed the body wall, the retrieval bag can then be reopened by manually grasping the closed end of the retrieval bag 20 and the bead and sliding the bead 50 along the cord 32. The retrieval bag 20 can then be accessed to remove or compact its contents to aid with complete withdrawal from the body cavity using standard open and endoscopic instrumentation such as forceps, graspers, and aspiration probes. Once the bulk of the contents are removed, the retrieval bag 20 can then be closed by manually grasping the open end of the retrieval bag 20 and the bead and sliding the bead 50 along the cord 32. The cord loop 32 can then be grasped manually and the retrieval bag 20 then completely withdrawn from the body cavity.

A second method for withdrawal of the retrieval bag 20 from within the body cavity can be used for small tissue specimens placed in the retrieval bag 20 which are not likely to need to be aspirated, compacted, or removed from the retrieval bag 20 prior to withdrawal of the retrieval bag 20 through the body wall. In this case, the cord loop 32 can be left attached to the actuator 7 and the entire device along with the trocar seal and cannula can be simultaneously withdrawn from the body cavity and through the body wall.

In a third method, the tissue retrieval systems described herein can be used in conjunction with a wound retractor such as an Alexis^{®} retractor, as discussed above. A method of using a tissue retrieval system in conjunction with a wound retractor includes first inserting a small wound retractor in the wound that would be lightly tensioned such as by inverting an external ring of the wound retractor to remove slack in the wound retractor sleeve. A trocar is then inserted within the sleeve of the wound retractor into the lined incision. A tissue retrieval system, such as one of the ones discussed herein would be inserted through the trocar and deployed. A tissue specimen is placed in the deployed bag. Once the specimen is placed in the tissue bag, the bag is cinched closed. In certain examples described herein, the cord loop can be detached from the actuator. With the bag cinched closed about a specimen contained therein, the introducer tube can be removed from the bag. In some examples, the trocar can also removed from the surgical site. The wound retractor is then fully tensioned to retract the wound to facilitate removal of the tissue bag from the body cavity such as by inverting an external ring to fully retract the incision site. With the incision site fully retracted, the retrieval bag described herein can be pulled through the wound retractor and out of the patient. Advantageously, a wound retractor can allow a tissue retrieval bag to be pulled through a relatively small incision without requiring the incision to be extended for passage of the bag therethrough.

The tissue retrieval bags 20 described herein can be utilized as stand alone retrieval bags that would be delivered to the surgical site through a trocar by rolling the retrieval bags and then pushing or pulling them through a trocar. A retrieval bag 20, in this aspect, would be provided with a cord loop 32 and can also be provided with a guide bead 50 or alternatively a slip knot for cinching the bag 20 closed. The reinforced distal section of the rim 26 of the retrieval bag 20 can allow the distal portion of the retrieval bag cuff 30 to remain in an open position without the need for metal or plastic supports. This version of the retrieval bag can be delivered through various trocars ranging in diameter from 5-15mm. The stand-alone retrieval bag 20 could be formed from thick laminate materials to enable the use of tissue morcellators to be utilized for reducing the size of large tissue specimens such as the spleen. The elongate profile of the retrieval bag 20 facilitates the insertion and use of a tissue morcellator within the retrieval bag and is particularly advantageous during single incision laparoscopic procedures. The elongate profile retrieval bag also enables the retrieval bag 20 to be delivered to and fit within confined surgical sites due to its low profile shape.

With reference to Figures 10-15, various examples of hybrid tissue retrieval bags 20" are illustrated. Rather than being formed of materials having substantially uniform properties extending along the retrieval bag between an open end and a closed end, the hybrid specimen retrieval bags have at least two portions with different properties (such as, for example, compliance, thickness, or number of layers of material), such that the bags are adapted for the clinical environment in which they are deployed. As discussed above with reference to Figures 7-8, a hybrid tissue retrieval bag 20' can have an elongate profile.

Tissue retrieval bags formed of a material with uniform properties throughout can create undesirable stress concentrations in use. Retrieval bags typically have a large diameter opening in the cuff portion to facilitate insertion of a specimen and a smaller diameter of the distal closed-end of the retrieval bag. An exemplary uniform tissue retrieval bag with an internal diameter of about 79mm (2.75") and a wall thickness of about 0.10mm (4 mil) in the cuff portion of the bag will experience a tensile stress of about 5.98MPa (867 PSI) in the cuff portion of the bag under tensile load of about 13.6Kg (30 pounds). The same retrieval bag with an internal diameter of about 19mm (0.75") and a wall thickness of about 0.10mm (4 mil) at the distal end of the bag will experience a stress of about 21.83MPa (3166 PSI) in the distal end of the bag under the same tensile load of about 13.6Kg (30 pounds). The induced tensile stresses of the uniform-material retrieval bag in the cuff portion and the lower distal portions are not balanced due to the differences in their respective diameters. The induced tensile stress of 5.98MPa (867 PSI) in the cuff portion of the bag is relatively low compared to the induced tensile stress of 21.83MPa (3166 PSI) in the distal end of the bag. Thus, a substantially uniform non-hybrid bag can require a relatively high strength material or a relatively thick material throughout to provide a desired strength at the distal end even when such a material is not required at the cuff portion of the bag. Accordingly, it can be desirable that a specimen retrieval bag be configured to generate a more balanced stress response under tensile loads expected in clinical application.

Thus, advantageously, the tissue retrieval system can include a retrieval bag configured to provide enhanced strength at stress concentrations versus prior art retrieval bags. The tissue retrieval system can include a "hybrid" retrieval bag 20" configured to provide localized areas of relatively high strength and localized areas of relatively lower strength and higher compliance. The hybrid retrieval bag can have localized areas of high strength in locations such as a distal, closed end and/or a distal end of the cuff, where tissue retrieval bags typically encounter relatively high stress. Figure 10 illustrates an exemplary hybrid tissue retrieval bag 20" in a tissue retrieval system 10' including a handle 9, an actuator rod 7, and an introducer tube 3 for insertion through a trocar.

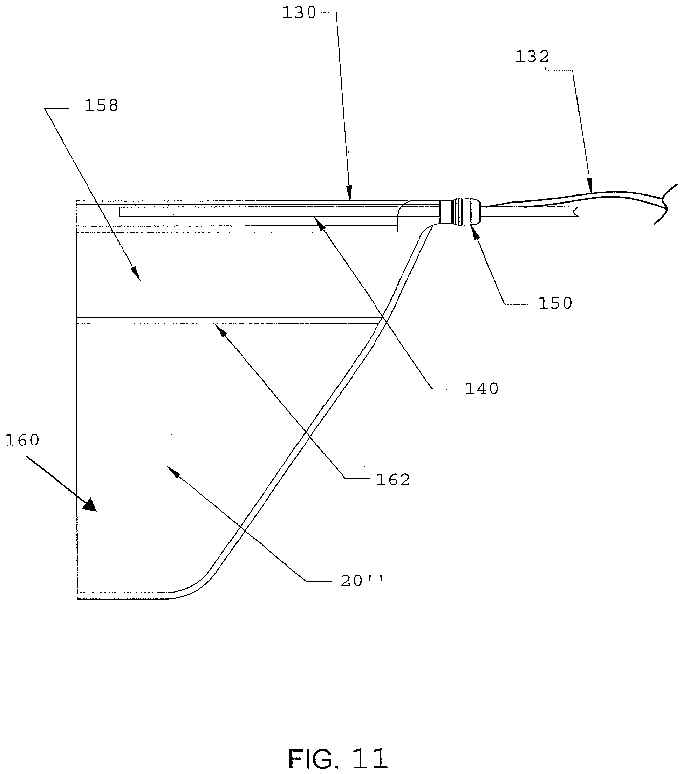

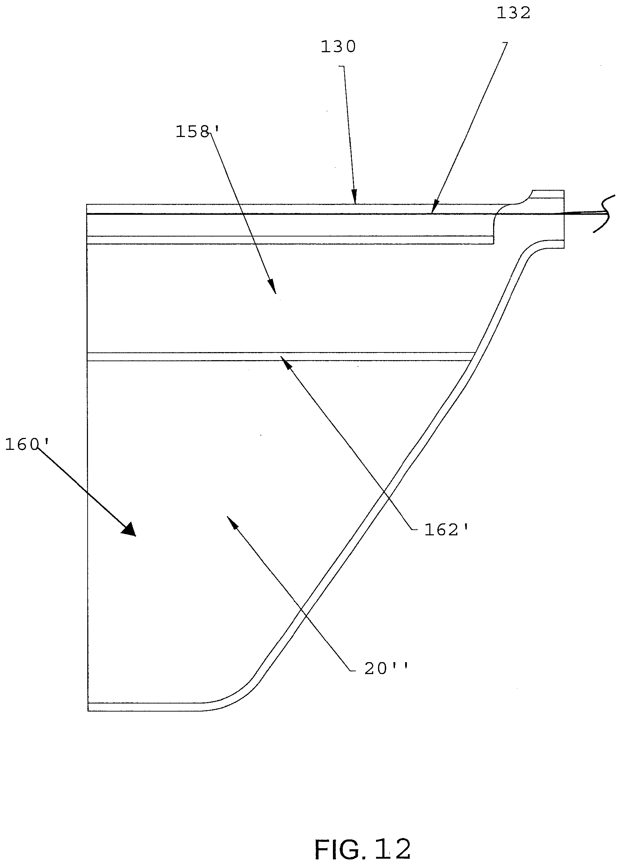

With reference to Figure 11, a hybrid retrieval bag 20" can be formed of at least two different film materials to provide relatively high flexibility at the proximal open end of the retrieval bag 20" and relatively high strength at the distal closed-end of the retrieval bag 20". With reference to Figure 12, a hybrid retrieval bag 20" can be formed with at least two different thicknesses of the same film material. The retrieval bag 20" can be formed with a relatively thin film in the proximal open end of the retrieval bag 20" to enhance flexibility while maintaining adequate strength and a relatively thick film in the distal closed-end of the retrieval bag 20" to enhance strength while enabling the retrieval bag 20" to be rolled and stored in the introducer tube 3.

Alternatively, a hybrid retrieval bag 20" can be formed with at least two different thicknesses of at least two different film materials. The hybrid retrieval bag 20" can provide flexibility at the open proximal end of the retrieval bag 20" and strength at the distal closed-end of the retrieval bag 20" while enabling the retrieval bag 20" to be easily rolled and stored in the introducer tube 3.

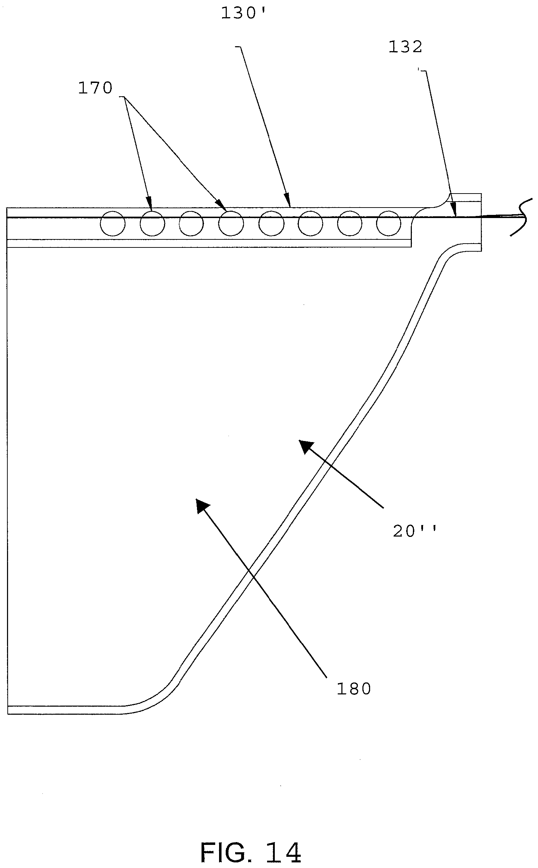

With reference to Figures 14-15, a hybrid retrieval bag 20" can be formed with one or more compliant features such as holes or slots in the cuff portion of the bag to provide the cuff with an increased degree of compliance. Here, a relatively stiff high-strength material can be used for forming the tissue retrieval bag 20".

One example of hybrid retrieval bag 20" formed of two film materials is illustrated in Figure 11. The hybrid retrieval bag 20" formed from two film materials is advantageous in that a first film material with a relatively high elongation and low modulus can be used for the upper cuff portion 158 of the retrieval bag 20" and a second film material with a relatively low elongation and a high modulus can be used for the lower, distal end portion 160 of the retrieval bag 20". A high modulus material requires a greater amount of force or pressure to be applied to the material to produce a given amount of elongation as compared to a low modulus material. Thus, advantageously, the tissue retrieval bag 20" will have relatively low elongation at the distal end portion 160, which, as discussed above, encounters relatively high tensile stress during clinical use.

By using a relatively high modulus film for the lower distal end portion 160 of the retrieval bag, the retrieval bag 20" will have a greater resistance to localized deformation and elongation than a comparable non-hybrid tissue retrieval bag. Thus, the hybrid configuration of the retrieval bag 20" contributes to the ability of the retrieval bag with a contained specimen to be easily withdrawn through the abdominal wall of the patient. The high modulus film material will not elongate as much as a low modulus film material and will therefore be less susceptible to deformation under the high tensile force that can be applied to the retrieval bag during withdrawal of the retrieval bag from that peritoneal cavity. Under the high tensile forces applied to the retrieval bag during withdrawal from the peritoneal cavity, a non-hybrid retrieval bag formed solely of low modulus film materials can significantly elongate and stretch until the retrieval bag deforms to take on a bulbous shape within the peritoneal cavity. Once a retrieval bag deforms to a bulbous shape, it can be difficult to remove the retrieval bag through the abdominal wall. In certain instances where a retrieval bag formed of a low modulus film has deformed, it can be required that the abdominal wall tract is enlarged to enable passage of the retrieval bag through the tract. In some cases, aspiration of some of the retrieval bag contents must be conducted with an irrigation/aspiration probe to enable passage of the retrieval bag through the abdominal wall tract.

Advantageously, a hybrid retrieval bag formed of a high modulus film material for the lower portion 160 of the retrieval bag 20" is less likely to deform than a comparable non-hybrid retrieval bag formed of a relatively low-modulus material. Furthermore, the hybrid retrieval bag 20" tends to force the contained specimen to elongate within the retrieval bag 20" and to conform to the shape of the retrieval bag 20" and thus facilitates withdrawal of the retrieval bag from the peritoneal cavity.

With continued reference to Figure 11, retrieval bag 20" formed from two film materials includes an upper cuff portion 158 formed of a first film material and a lower, distal end portion 160 formed of a second film material having a different compliance and a different modulus than the first film material. The upper cuff portion 158 has a depth toward the closed end of the bag that measures about 38mm (1.5") from the open end of the retrieval bag 20". The upper cuff portion 158 can have different depths. For example, the upper cuff portion can have a depth within the range of about 12.7mm (one half an inch) to about 76mm (three inches), alternatively, the upper cuff portion can have a depth between about 25mm (one inch) and about 63mm (two and a half inches), or the upper cuff can have a depth of between about 32mm (one and a quarter inches) and 44mm (one and three quarters inches).

The retrieval bag 20" can have a total depth between the open end and the closed end of approximately 127mm (5 inches), but it can be desirable to have a retrieval bag with a total depth of less than 127mm (5 inches) or greater than 127mm (5 inches). Accordingly, to account for dimensional differences in different sized bags, it can be useful to describe the depth of the upper cuff portion 158 in terms of a ratio with the total depth of the tissue retrieval bag 20". The ratio of the depth of the upper cuff portion to the depth of the bag from the open end to the closed end can be between about 1:10 and about 1:1.5, in other examples the ratio of the depth of the upper cuff of the tissue retrieval bag to the total depth of the beg is between about 1:4 and about 1:1.8, and in other examples the ratio of the depth of the upper cuff of the retrieval bag to the total depth of the tissue retrieval bag is between about 1:3 and about 1:2.

The first film material, comprising the upper cuff portion 158 comprises a 0.10mm (.004" or 4 mil) polyurethane film material, although in different thicknesses of film can be used, such as, for example, a 0.05mm (2 mil) film, 0.15mm (6 mil) film, or film of another suitable thickness. Furthermore, the upper cuff portion 158 comprises a polyurethane material, but the first film material could comprise another suitable film material.

Film material properties are typically reported as modulus at 100% elongation of the film with modulus in the units of pounds per square inch or pascals. As illustrated, the first film material comprising the upper cuff portion 158 has a modulus of about 6.55MPa (950 PSI) at 1000% elongation. However, the modulus can be between about 3.45MPa (500 PSI) a 100% elongation and about 8.96MPa (1300 PSI) at 100% elongation. Or, the modulus can be between 5.17MPa (750 PSI) at 100% elongation and about 7.58MPa (1100 PSI) at 100% elongation. Or, the modulus can be between about 6.21MPa (900 PSI) at 100% elongation and about 6.89MPa (1000 PSI) at 100% elongation.

With continued reference to Figure 11, the lower, distal or closed-end portion 160 of the retrieval bag 20" is formed of a second film material having a second compliance or modulus. As illustrated, the distal-end portion 160 of the retrieval bag 20" has a depth measuring about 89mm (3.5") from the interface or joint 162 with the upper cut portion 158 of the retrieval bag to the closed end of the bag 20". In other examples, the distal-end portion of the bag can have a depth of between about 25mm (1 inch) to about 114mm (4½ inches), the distal end portion of the bag 20" could have a depth between about 63mm (2½ inches) and about 102mm (4 inches), and in other examples, the distal end portion of the bag can have a depth between about 83mm (3¼ inches) and about 95mm (3¾ inches).

In terms of a ratio of depth of the distal end portion 160 of the bag 20" to the total depth of the bag 20" between the open end and the closed end, , the ratio of the depth of the lower portion of the bag 20" to the total depth is between about 1:3 and about 1:1.1.

The second film material comprises a 0.10mm (.004 inches) polyurethane film material.

As illustrated, the second film material comprising the distal end portion 160 of the retrieval bag 20" has a modulus of about 11.03MPa (1600 PSI) at 100% elongation.

The relatively low modulus material in the cuff portion 158 of the retrieval bag 20" can allow the bag 20" to be easily opened and closed while the relatively high modulus material in the lower distal-end portion 160 of the retrieval bag 20" can reduce or substantially prevent elongation of the retrieval bag and force the specimen itself to take on the shape of the retrieval bag. Thus, the described hybrid retrieval bag 20" can provide eased withdrawal of the retrieval bag from the body cavity as compared to a comparable non-hybrid bag.

With reference to Figure 12, other examples of hybrid retrieval bag 20" are illustrated including at least two portions having film material of different thicknesses. A retrieval bag 20" formed with two different thicknesses of film material can be advantageous in that the cuff portion 158' of the retrieval bag 20" can be formed of a smaller gauge film than in a distal-end portion 160' of the retrieval bag 20". Thus, the cuff portion 158' of the retrieval bag is relatively compliant compared to the distal-end portion 160'. The strength and puncture resistance of the distal-end portion 160' of the retrieval bag 20" can be enhanced by utilizing a relatively larger gauge film. A larger gauge film can be used in the distal-end portion 160' of the retrieval bag than would otherwise be possible if the retrieval bag with the same shape and size were to be manufactured entirely from a uniform thickness film. By incorporating a relatively thin film in the cuff portion 158' of the retrieval bag and a relatively thick film in the distal-end portion 160' of the retrieval bag 20", the volume of the bag when rolled and stored in the introducer tube 3 can be substantially equivalent to the volume of retrieval bag formed with a uniform film thickness. But, the hybrid retrieval bag 20" can have a significantly greater strength in the distal-end portion 160' of the retrieval bag (where strength can be highly desirable) than a comparable non-hybrid uniform film thickness bag.

The distal-end portion 160' of the retrieval bag 20" has a circumference which is significantly less than a circumference of the cuff portion 150' of the retrieval bag. As discussed above, for a retrieval bag formed from a uniform thickness film, the stress transferred to the cuff portion of the retrieval bag during withdrawal of the bag from a patient by cinching the cord loop 132 through the cuff 130 is significantly less than the stress transferred to the lower distal portion of the tissue bag. Thus, a thin gauge film can be used in the cuff portion 158' of the retrieval bag to provide a desired tensile strength while a thick gauge film can be used in the distal-end portion 160' of the retrieval bag 20" to provide increased tensile strength, increased hoop strength, and increased puncture resistance. These improvements can be obtained in hybrid tissue retrieval bags 20" using two thicknesses of the same film material or two thicknesses of different film materials.

With continued reference to Figure 12, hybrid retrieval bag 20" can have a cuff portion 158' with an internal diameter of about (800mm (2.75") and a wall thickness of about 0.05mm (2 mil) in the cuff portion. Accordingly, the cuff portion 158' of retrieval bag 20" will experience a tensile stress of about 11.96MPa (1735 PSI) under a tensile load of about 13.6 Kg (30 pounds).

The same retrieval bag 20" can have an internal diameter of about 19mm (.75") and a wall thickness of about 0.15mm (6 mil) at a distal end of the bag. Accordingly, the distal end portion of the retrieval bag will experience a tensile stress of about 14.51MPa (2105 PSI) under a tensile load of about 13.6 Kg (30 pounds). Thus, advantageously, the tensile stresses encountered by the illustrated tissue retrieval bag 20" are substantially more balanced as compared with the exemplary comparable uniform, non-hybrid tissue retrieval bag described above.

With continued reference to Figure 12, while the retrieval bag 20" includes a cuff portion 158' having a 0.05mm (2 mil) film thickness and a distal end portion 160' having a 0.15mm (6 mil) film thickness, it is contemplated that in other other film thicknesses can be used. For example, the cuff portion can have a film thickness of between about 0.025mm (1 mil) and about 0.13mm (5 mil). The distal portion can have a film thickness greater than that of the cuff portion and between about 0.08mm (3 mil) and about 0.23mm (9 mil).

Advantageously, a hybrid retrieval bag 20" with varying wall thicknesses, can have substantially balanced stresses between the cuff portion 158' and the distal-end portion 160'. Thus, a hybrid retrieval bag can be configured to provide a desired strength at the distal end while maintaining compliance at the cuff for installation in an inserter tube. The material thicknesses of the cuff portion and distal portion of the hybrid retrieval bag can be chosen such that for a predetermined tensile load, the corresponding tensile stresses are equal.

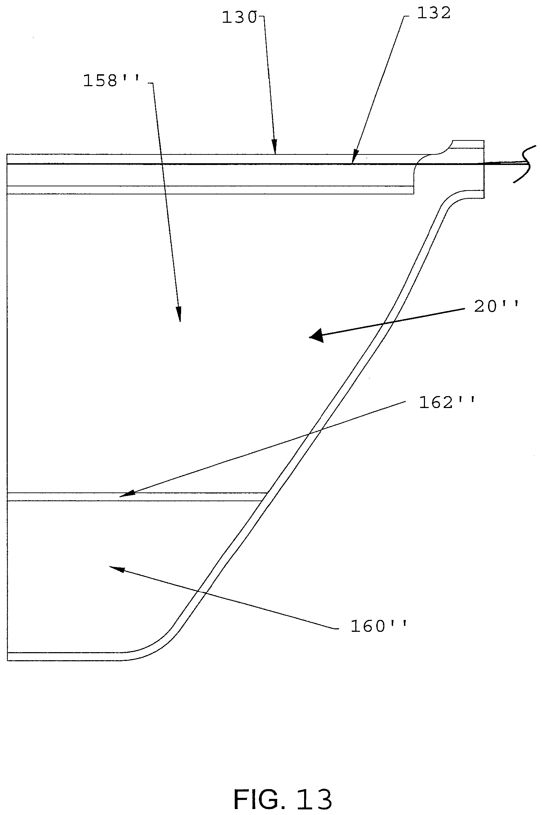

With reference to Figure 13, the retrieval bag 20" can be formed with a reinforced wall segment in the distal-end portion 160" of the bag. As discussed above, during withdrawal of the retrieval bag 20" through a body wall, the specimen is typically forced into the distal-end portion 160" of the bag, subjecting this portion of the retrieval bag to high stresses. The distal portion of the retrieval bag typically has the least cross-sectional area for supporting a tensile load and it is therefore advantageous to enhance the strength of the distal portion of the retrieval bag. Thus, it can be advantageous to increase the strength of the distal-end portion 160" of the retrieval bag by providing a reinforced section, such as a multiple layer wall section. The reinforced section comprises a double wall configuration in the distal-end portion 160" of the retrieval bag 20". Advantageously, by providing a reinforced section in only the distal-end portion 160" of the retrieval bag 20", the tensile strength of the distal-end portion 160" of the bag 20" can be relatively increased while maintaining the rolled volume of the retrieval bag 20" at a relatively small diameter compared to a non-hybrid retrieval bag having equivalent tensile strength at the distal end. Thus, the hybrid retrieval bag 20" can be rolled and stored in a relatively low-diameter introducer tube 3 as compared to a non-hybrid retrieval bag having equivalent tensile strength at the distal end.

In some examples, the hybrid retrieval bag 20" with a reinforced section can be reinforced with a very stiff high modulus, high-strength material that might not otherwise be suitable on its own for manufacturing a retrieval bag. For example in, a retrieval bag formed of a polyurethane film can be reinforced with a fabric material such as a non-coated rip stop nylon to prevent the distal portion of the retrieval bag from deforming during withdrawal from a body cavity.

The reinforced section of the distal-end portion 160" can be coupled to the hybrid retrieval bag 20" in various ways. For example, the reinforced section can be chemically adhered to the retrieval bag with a bio-compatible chemical adhesive, thermally fused, ultrasonically welded, overmolded, dip molded or sewn onto the retrieval bag. The hybrid specimen retrieval bags 20" illustrated in Figures 11-12 can be formed with similar processes for joining the cuff portions 158, 158' to the distal-end portions 160, 160', as discussed with respect to the specimen retrieval bags 20" of Figure 13, and can each include a joint 162, 162' between the corresponding cuff portions 158, 158' and distal-end portions 160, 160'.

With continued reference to Figure 13, a hybrid retrieval bag 20" with a reinforced section can have an external joint 162" such as a weld or seam extending along an outer surface of the tissue retrieval bag between the reinforced section of the distal-end portion 160" and the cuff portion 158" of the tissue retrieval bag 20"

The reinforced section can be coupled to the distal portion of the retrieval bag 20" over substantially an entire inner surface of the reinforced section. For example, the reinforced section' can comprise a laminate of two layers of film material.

With reference to Figures 14-15, the tissue retrieval bag 20" can have a compliant cuff 130', 130" through which a cord loop 132 passes and a bag portion 180 extending from the cuff 130', 130" to the closed end of the bag 20". The cuff 130', 130" can be relatively compliant relative to the bag portion 180. The bag portion 180 can comprise an upper bag portion and a lower bag portion, each having different properties (for example, the bag portion 180 can be similar to the upper cuff portion and lower distal end portion of one of the hybrid retrieval bags described with respect to Figures 11-13 with the compliant cuff 130', 130" desirably being more compliant than an upper cuff portion of the hybrid retrieval bag 20" described with respect to Figures 11-13).

The cuff 130', 130" can include various compliance enhancing features. Increasing the compliance of the cuff 130', 130" facilitates the opening and closure of the retrieval bag 20" and enables retrieval bag 20" to be formed from a film material with a greater thickness than would otherwise be possible in a comparable non-hybrid retrieval bag. Increasing the compliance of the cuff also enables retrieval bag to be formed from a material with a higher modulus than would otherwise be possible. Increasing the retrieval bag material modulus and/or thickness improves the tensile strength of the retrieval bag.

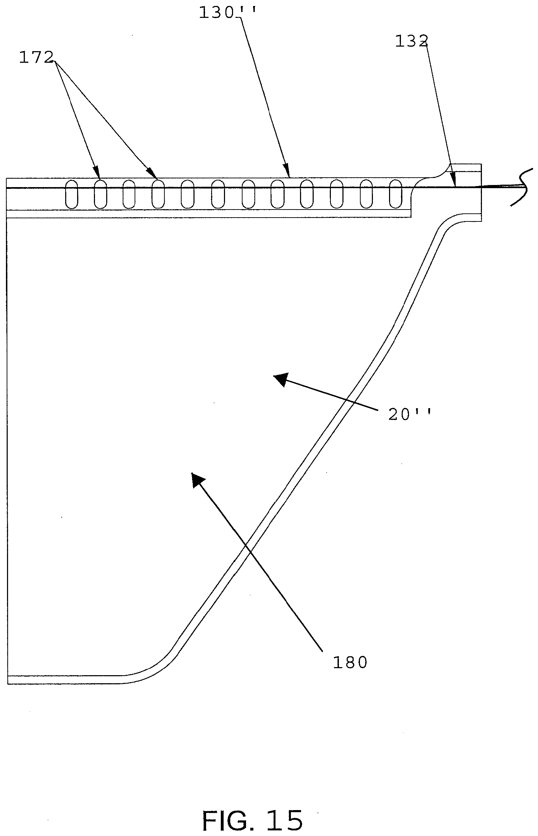

The cuff 130', 130" can include one or more of various compliance enhancing features such as holes 170 (Figure 14), slots 172 (Figure 15), slits, or notches in the cuff to provide the cuff with increased compliance. The compliance enhancing features can be die cut into an otherwise solid cuff during manufacture of the retrieval bag.

In one method of manufacture, the cuff of the retrieval bag 20" can be formed by folding the film at the open end of the retrieval bag over on itself and joining the film to itself such as by an adhesive bonding, thermal welding, or ultrasonic welding process. This folded and joined segment of film forms a cuff channel bounded by two film walls. A closure member and retrieval bag supports can pass through the cuff channel. As described above, in use, after the retrieval bag supports are withdrawn from the cuff channel, the cord loop is then tensioned and the cuff channel is forced to fold longitudinally thereby cinching the bag closed. The holes, slots, slits, or notches can be formed through one or both of the walls of the cuff channel to provide a desired degree of compliance.

The compliance enhancing features can be provided circumferentially throughout the peripheral extent of the cuff or over a portion of the peripheral extent of the cuff. For example, in Figures 14 and 15, a distal tip region of the cuff has no compliance enhancing features. Compliance enhancing features can be provided throughout a depth of the cuff of the retrieval bag or over a portion of the depth of the cuff of the retrieval bag. Compliance enhancing features can be of uniform size throughout the cuff portion or can vary in size along the cuff. For example, at least some of the compliance enhancing features can be sized to prevent the retrieval bag supports from protruding through the wall of the cuff during deployment of the retrieval bag from the introducer tube.

While as discussed above with respect to Figure 13, the hybrid retrieval bag 20" can include a reinforced section at the distal-end portion 160" of the bag 20", any of the retrieval bags described herein the retrieval bag can be reinforced along the axis of the cord loop length and/or transverse to the axis of the cord loop to minimize the elongation of the bag during withdrawal from the body cavity. For example, a mesh material, strips of a film material, strips of a woven fabric, cord, or wire can be attached to the retrieval bag to reinforce the bag and prevent elongation and deformation of the bag.

While as discussed above with respect to Figure 11, the retrieval bag 20" can be formed of two portions 158, 160 of material having different properties, the retrieval bag could be formed of more than two portions of material each having different properties. For example, Figure 11 illustrates a bag having a first material for the cuff portion with a relatively high compliance and a second material for the distal portion having a relatively low compliance. Alternatively, the retrieval bag can be formed from three or more different film materials such that the cuff portion is formed of a low modulus, highly flexible material, an intermediate portion is formed of a media modulus, moderately flexible material, and the distal portion could be formed of a high modulus, relatively inflexible material.

Additionally, while Figure 12 illustrates a tissue retrieval bag having two portions of material having different thicknesses, a retrieval bag could be formed from three or more portions of material each having different thicknesses. For example, the cuff portion could formed of a thin material, such as a 0.05mm (2 mil) thick film, the intermediate portion is formed of a moderately thick material such as a 0.15mm (6 mil) thick film, and the distal portion is formed of a relatively thick material such as a 0.20mm (8 mil) thick film.

Moreover, while Figure 13 illustrates a retrieval bag having a reinforced section with an additional layer of film material at the distal end, a retrieval bag can have an intermediate portion having an intermediate level of reinforcement such as two layers of film material and relatively highly reinforced section at a distal end portion such as with three or more layers of film material.

A hybrid retrieval bag could be formed of a single film material which progressively increases in wall thickness from the proximal open end of the retrieval bag to the distal closed-end of the retrieval bag. Here, the retrieval bag can advantageously have a relatively high strength at the closed end and a relatively high compliance at the open end.

As discussed above a tissue retrieval system having a hybrid tissue retrieval bag can be manufactured in a range of sizes and configurations to accommodate varying clinical needs.

Although this application discloses certain preferred embodiments and examples, other alternative embodiments and/or uses, modifications and equivalents thereof will be apparent to those skilled in the art, that may fall within the scope of the invention as defined by the following claims.

## Claims

1. A tissue retrieval system (10) comprising:
an introducer (3) having a proximal end, a distal end, and a lumen formed therein extending from the proximal end to the distal end;
a handle assembly (5) extending from the proximal end of the introducer (3);
an actuator rod (7) having a proximal end and a distal end, at least a portion of the actuator rod (7) being slidable within the lumen of the introducer (3);
two support arms (40) extending from the distal end of the actuator rod (7);
a tissue retrieval bag (20) deployable by the actuator rod (7), the tissue retrieval bag (20) having a closed distal end (24) opposite a proximal end (22) and having a rim (26) defining an opening adjacent the proximal end (22), with the tissue retrieval bag having an elongate profile, with a portion of the tissue retrieval bag extending distally from the rim (26) and the opening, wherein the actuator rod (7) is slidable relative to the introducer (3) from a first position in which the tissue retrieval bag (20) is in a stowed configuration to a second position in which the tissue retrieval bag (20) is in a deployed configuration, wherein the support arms (40) are biased to a predetermined position when the tissue retrieval bag (20) is in the deployed configuration and wherein, in the deployed configuration, the tissue retrieval bag (20) is suspended and held open by the two support arms (40) extending into the cuff (30) of the tissue retrieval bag (20);
a cord loop (32) for cinching the retrieval bag closed and for withdrawing the retrieval bag (20) through a body wall;
**characterized in that** the tissue retrieval bag (20) is formed from a flat sheet of film material and has a non-continuous cuff (30) adjacent the opening, wherein the cuff is formed by folding a portion of the film material over itself and joining the folded portion to the remainder of the material and wherein the two support arms (40) are receivable in the non-continuous cuff (30) and the cord loop (32) extends through the non-continuous cuff (30).

2. The tissue retrieval system of claim 1, wherein the tissue retrieval bag (20) is formed from a ripstop nylon material.

3. The tissue retrieval system of Claim 1, wherein the tissue retrieval bag (20) is formed from one of a polyurethane coated ripstop nylon material and a silicone coated ripstop nylon material.

4. The tissue retrieval system of any of the preceding claims, wherein upon deployment of the tissue retrieval bag, the support arms (40) are biased to spring radially outward and transversely upward relative to a longitudinal axis extending between the proximal end and the distal end of the actuator rod (7).

5. The tissue retrieval system of any of the preceding claims, wherein the support arms (40) have a curved profile upon deployment of the tissue retrieval bag.

6. The tissue retrieval system of any of the preceding claims, wherein the tissue retrieval bag (20) further comprises a reinforcing tab (38) positioned to reinforce the non-continuous cuff.

7. The tissue retrieval system of claim 6, wherein the reinforcing tab (38) is formed from a segment of a film material having an hourglass shape.

8. The tissue retrieval system of any of the preceding claims, wherein the two support arms (40) each comprise a spring metal material.

## Patentansprüche

1. Gewebeentnahmesystem (10), das Folgendes umfasst:
ein Einführelement (3), das Folgendes aufweist: ein proximales Ende, ein distales Ende und ein Lumen, das darin ausgebildet ist, das sich von dem proximalen Ende zu dem distalen Ende erstreckt;
eine Griffanordnung (5), die sich von dem proximalen Ende des Einführelements (3) erstreckt;
eine Betätigungsstange (7), die ein proximales Ende und ein distales Ende aufweist, wobei zumindest ein Teil der Betätigungsstange (7) innerhalb des Lumens des Einführelements (3) verschiebbar ist;
zwei Haltearme (40), die sich von dem distalen Ende der Betätigungsstange (7) erstrecken;
einen Gewebeentnahmebeutel (20), der durch die Betätigungsstange (7) einsetzbar ist, wobei der Gewebeentnahmebeutel (20) ein geschlossenes distales Ende (24) gegenüber einem proximalen Ende (22) aufweist und einen Rand (26) aufweist, der eine Öffnung benachbart zu dem proximalen Ende (22) definiert, wobei der Gewebeentnahmebeutel ein längliches Profil aufweist, wobei sich ein Teil des Gewebeentnahmebeutels distal von dem Rand (26) und der Öffnung erstreckt, wobei die Betätigungsstange (7) in Bezug auf das Einführelement (3) von einer ersten Position, in der der Gewebeentnahmebeutel (20) in einer verstauten Konfiguration vorliegt, zu einer zweiten Position, in der der Gewebeentnahmebeutel (20) in einer eingesetzten Konfiguration vorliegt, verschiebbar ist, wobei die Haltearme (40) zu einer vorgegebenen Position vorgespannt sind, wenn der Gewebeentnahmebeutel (20) in einer eingesetzten Konfiguration vorliegt, und wobei in der eingesetzten Konfiguration der Gewebeentnahmebeutel (20) aufgehängt ist und durch die zwei Haltearme (40), die sich in den Umschlag (30) des Gewebeentnahmebeutels (20) erstrecken, offen gehalten wird;
eine Schnurschlaufe (32) zum Zusammenziehen des Entnahmebeutels und zum Zurückziehen des Entnahmebeutels (20) durch eine Körperwand;
**dadurch gekennzeichnet, dass** der Gewebeentnahmebeutel (20) aus einer flachen Lage von Folienmaterial ausgebildet ist und einen nicht-fortlaufenden Umschlag (30) benachbart zur Öffnung aufweist, wobei der Umschlag durch Falten eines Teils des Folienmaterials über sich selbst und Verbinden des gefalteten Teils zu dem Rest des Materials ausgebildet wird und wobei die zwei Haltearme (40) in dem nicht-fortlaufenden Umschlag (30) aufnehmbar sind und sich die Schnurschlaufe (32) durch den nicht-fortlaufenden Umschlag (30) erstreckt.

2. Gewebeentnahmesystem nach Anspruch 1, wobei der Gewebeentnahmebeutel (20) aus einem Ripstop-Nylonmaterial ausgebildet ist.

3. Gewebeentnahmesystem nach Anspruch 1, wobei der Gewebeentnahmebeutel (20) aus einem eines Polyurethan-beschichteten Ripstop-Nylonmaterials und eines Silikon-beschichteten Ripstop-Nylonmaterials ausgebildet ist.

4. Gewebeentnahmesystem nach einem der vorstehenden Ansprüche, wobei bei Einsatz des Gewebeentnahmebeutels die Haltearme (40) vorgespannt sind, um radial nach außen und quer nach oben in Bezug auf eine Längsachse, die sich zwischen dem proximalen Ende und dem distalen Ende der Betätigungsstange (7) erstreckt, zu springen.

5. Gewebeentnahmesystem nach einem der vorstehenden Ansprüche, wobei die Haltearme (40) ein gebogenes Profil bei Einsatz des Gewebeentnahmebeutels aufweisen.

6. Gewebeentnahmesystem nach einem der vorstehenden Ansprüche, wobei der Gewebeentnahmebeutel (20) weiter einen Verstärkungsstreifen (38) umfasst, der zur Verstärkung des nicht-fortlaufenden Umschlags positioniert ist.

7. Gewebeentnahmesystem nach Anspruch 6, wobei der Verstärkungsstreifen (38) aus einem Segment eines Folienmaterials, das eine Sanduhrform aufweist, ausgebildet ist.

8. Gewebeentnahmesystem nach einem der vorstehenden Ansprüche, wobei die zwei Haltearme (40) jeweils ein Metallfedermaterial umfassen.

## Revendications

1. Système d'extraction de tissus (10) comprenant :
un introducteur (3) ayant une extrémité proximale, une extrémité distale et une lumière formée dedans s'étendant de l'extrémité proximale à l'extrémité distale ;
un ensemble de poignée (5) s'étendant de l'extrémité proximale de l'introducteur (3) ;
une tige d'actionneur (7) ayant une extrémité proximale et une extrémité distale, au moins une partie de la tige d'actionneur (7) pouvant glisser dans la lumière de l'introducteur (3) ;
deux bras de support (40) s'étendant de l'extrémité distale de la tige d'actionneur (7) ;
un sac d'extraction de tissus (20) déployable par la tige d'actionneur (7), le sac d'extraction de tissus (20) ayant une extrémité distale fermée (24) à l'opposé d'une extrémité proximale (22) et ayant un rebord (26) définissant une ouverture adjacente à l'extrémité proximale (22), avec le sac d'extraction de tissus ayant un profil allongé, avec une partie du sac d'extraction de tissus s'étendant distalement du rebord (26) et de l'ouverture, dans lequel la tige d'actionneur (7) peut glisser par rapport à l'introducteur (3) d'une première position dans laquelle le sac d'extraction de tissus (20) est dans une configuration repliée à une deuxième position dans laquelle le sac d'extraction de tissus (20) est dans une configuration déployée, dans lequel les bras de support (40) sont sollicités vers une position prédéterminée lorsque le sac d'extraction de tissus (20) est dans la configuration déployée et dans lequel, dans la configuration déployée, le sac d'extraction de tissus (20) est suspendu et tenu ouvert par les deux bras de support (40) s'étendant dans le revers (30) du sac d'extraction de tissus (20) ;
un cordon de serrage (32) pour sangler fermé le sac d'extraction et pour retirer le sac d'extraction (20) à travers une paroi corporelle ;
**caractérisé en ce que** le sac d'extraction de tissus (20) est formé d'une feuille plate de matériau en film et a un revers non continu (30) adjacent à l'ouverture, dans lequel le revers est formé en repliant une partie du matériau en film sur lui-même et en joignant la partie repliée au reste du matériau et dans lequel les deux bras de support (40) peuvent être reçus dans le revers non continu (30) et le cordon de serrage (32) s'étend à travers le revers non continu (30).

2. Système d'extraction de tissus selon la revendication 1, dans lequel le sac d'extraction de tissus (20) est formé d'un matériau anti-déchirure en nylon.

3. Système d'extraction de tissus selon la revendication 1, dans lequel le sac d'extraction de tissus (20) est formé de l'un d'entre un matériau en nylon anti-déchirure revêtu de polyuréthane et d'un matériau en nylon anti-déchirure revêtu de silicone.

4. Système d'extraction de tissus selon l'une quelconque des revendications précédentes, dans lequel lors du déploiement du sac d'extraction de tissus, les bras de support (40) sont sollicités pour se détendre radialement vers l'extérieur et transversalement vers le haut par rapport à un axe longitudinal s'étendant ente l'extrémité proximale et l'extrémité distale de la tige d'actionneur (7).

5. Système d'extraction de tissus selon l'une quelconque des revendications précédentes, dans lequel les bras de support (40) ont un profil recourbé lors du déploiement du sac d'extraction de tissus.

6. Système d'extraction de tissus selon l'une quelconque des revendications précédentes, dans lequel le sac d'extraction de tissus (20) comprend en outre une languette de renfort (38) positionnée pour renforcer le revers non continu.

7. Système d'extraction de tissus selon la revendication 6, dans lequel la languette de renfort (38) est formée d'un segment d'un matériau en film ayant une forme de sablier.

8. Système d'extraction de tissus selon l'une quelconque des revendications précédentes, dans lequel les deux bras de support (40) comprennent chacun un matériau en métal à ressort.
